# EUROPÄISCHE PATENTSCHRIFT

(11) **EP 1 888 132 B1**
(45) Veröffentlichungstag und Bekanntmachung des Hinweises auf die Patenterteilung: **12.08.2015**
(21) Anmeldenummer: 06724500.1
(22) Anmeldetag: 21.04.2006
(51) Int. Cl.: A61L 15/60, C08F 8/44, C08J 3/24

(54) **OBERFLÄCHENNACHVERNETZTE SUPERABSORBER BEHANDELT MIT WASSERLÖSLICHEM ALUMINIUMSALZ UND ZINKOXID**
SUPERABSORBER POST-RETICULATED ON THE SURFACE AND TREATED WITH A WATER SOLUBLE ALUMINIUM SALT AND ZINC OXIDE
SUPERABSORBANT RETICULE POSTERIEUREMENT A LA SURFACE ET TRAITE AVEC UN SEL HYDROSOLUBLE D'ALUMINIUM ET DE L'OXIDE DE ZINC

(30) Priorität: 22.04.2005 DE 102005018923; 27.12.2005 DE 102005062831
(43) Veröffentlichungstag der Anmeldung: 20.02.2008
(73) Patentinhaber: Evonik Degussa GmbH, 45128 Essen (DE)
(72) Erfinder: WALDEN, Mirko, 45699 Herten (DE); FURNO, Franck, 47799 Krefeld (DE); SCHMIDT, Harald, 47918 Tönisvorst (DE)
(74) Vertreter: Lang, Arne
(86) Internationale Anmeldenummer: PCT/EP2006/003696
(87) Internationale Veröffentlichungsnummer: WO 2006/111404

(56) Entgegenhaltungen:
- WO-A-2004/037900
- WO-A2-2004/037903
- DE-A1- 10 138 630
- DE-A1- 19 909 653
- US-A- 4 587 308
- US-A- 5 973 042
- US-A1- 2003 125 684

## Beschreibung

Die vorliegende Erfindung betrifft wasserabsorbierende Polymergebilde, ein Verfahren zur Behandlung der Oberfläche wasserabsorbierender Polymergebilde, die durch dieses Verfahren erhältlichen oberflächenbehandelten, wasserabsorbierenden Polymergebilde, einen Verbund beinhaltend ein wasserabsorbierendes Polymergebilde sowie ein Substrat, ein Verfahren zur Herstellung eines Verbundes, den durch dieses Verfahren erhältlichen Verbund, chemische Produkte wie Schäume, Formkörper und Fasern beinhaltend wasserabsorbierende Polymergebilde oder einen Verbund, die Verwendung wasserabsorbierender Polymergebilde oder eines Verbundes in chemischen Produkten sowie die Verwendung einer Kombination aus einem Oxid eines Metalls und eines Metallsalzes zur Behandlung der Oberfläche superabsorbierender Polymergebilde.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikeln, wie beispielsweise Babywindeln, Inkontinenzprodukten oder Damenbinden.

Die Herstellung der Superabsorber erfolgt in der Regel durch die radikalische Polymerisation säuregruppen-tragender Monomere in Gegenwart von Vernetzern. Dabei lassen sich durch die Auswahl der Monomerzusammensetzung, der Vemetzer sowie der Polymerisationsbedingungen und der Verarbeitungsbedingungen für das nach der Polymerisation erhaltene Hydrogel Polymere mit unterschiedlichen Absorbereigenschaften herstellen. Weitere Möglichkeiten bietet die Herstellung von Pfropfpolymerisaten, beispielsweise unter Verwendung chemisch modifizierter Stärke, Cellulose und Polyvinylalkohol nach DE-OS 26 12 846.

DE 40 20 780 C1 offenbart die Nachbehandlung superabsorbierender Polymere durch Nachvernetzung der Oberflächen der Polymerteilchen. Durch die Nachvernetzung der Oberfläche der wasserabsorbierenden Polymerteilchen wird insbesondere das Absorptionsvermögen der Polymerteilchen unter der Einwirkung von Drücken erhöht.

DE 199 09 653 A1 und DE 199 09 838 A1 beschreiben pulverförmige, an der Oberfläche nachvernetzte, Wasser, wässrige oder seröse Flüssigkeiten oder Blut absorbierende Polymerisate, welche auf säuregruppen-tragenden Monomeren basieren und welche mit einem Oberflächennachvernetzungsmittel und einem Kation in wässriger Lösung beschichtet und nachvernetzt worden ist. Die in diesem Stand der Technik offenbarten Polymerisate weisen gegenüber herkömmlichen Polymerisaten vorteilhafte Absorptionseigenschaften, insbesondere eine hohe Permeabilität auf.

DE 10138630 A1 offenbart Verfahren zur Herstellung von Rein-(Meth)acrylsäure und Methacrylsäureestern, welche in Superabsorbern Anwendung finden. Die dort beschriebenen Polymere können nachvernetzt werden und optional zur Modifizierung der Hydrophilie bzw. zur Verstärkung der Effekte der Oberflächenbehandlung mit Metallsalzen nachbehandelt werden.

Die in dem vorstehend zitierten Stand der Technik offenbarte Oberflächennachvernetzung der Polymergebilde führt zwar zu einer Erhöhung der Absorptionsfähigkeit unter einer Druckbelastung, jedoch ist mit dieser Oberflächennachvernetzung in der Regel einer Verminderung der Retentionskapazität verbunden. Umgekehrt führt eine beispielsweise durch geringe Primärvernetzung bewirkte Erhöhung der Retentionskapazität zu einem verminderten Absorptionsvermögen der Polymergebilde unter einer Druckbelastung. Eine verminderte Retentionskapazität der Polymergebilde hat jedoch den Nachteil, dass Hygieneartikel wie etwa Windeln, welche diese Polymergebilde beinhalten, durch ein vermindertes Aufnahmeund Rückhaltevermögen von Körperflüssigkeiten gekennzeichnet sind, während eine verminderte Absorption unter Druck insbesondere dazu führt, dass die Hygieneartikel Flüssigkeiten unter einer Druckbelastung, wie sie beispielsweise durch einen sitzenden Windelträger verursacht wird, nicht ausreichend aufnehmen können.

Polymergebilde, welche ein gutes Retentionsverhalten und ein gutes Absorptionsvermögen unter einer Druckbelastung in sich vereinigen, könnten möglicherweise dadurch erhalten werden, dass von vornherein nur geringfügig vernetzte Polymerpartikel, welche aufgrund des geringen Vernetzungsgrades eine vergleichsweise hohe Retention aufweisen, einer Oberflächennachvernetzung unterzogen werden. Dieses hat jedoch den Nachteil, dass die auf diese Weise erhaltenen Superabsorber durch einen hohen Gehalt an löslichen Anteilen gekennzeichnet sind, da der Gehalt an löslichen Anteilen mit abnehmendem Vernetzungsgrad zunimmt. Ein zunehmender Gehalt an löslichen Anteilen ist jedoch mit einer verminderten Hautverträglichkeit verbunden, so dass Hygieneartikel beinhaltend Polymergebilde mit einem hohen Gehalt an löslichen Anteilen aus dermatologischer Sicht bedenklich sind. Um den Gehalt an löslichen Anteilen in den Polymergebilden möglichst gering zu halten, ist daher ein Mindestmaß an Primärvernetzung im Polymergebilde erstrebenswert.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, Superabsorber bereitzustellen, die neben ausgezeichneten Retentionseigenschaften auch durch gute Absorptionseigenschaften unter einer Druckbelastung sowie vorteilhafterweise auch durch eine gute Hautverträglichkeit gekennzeichnet sind.

Weiterhin lag der vorliegenden Erfindung die Aufgabe zugrunde, ein Verfahren anzugeben, mit dem derart vorteilhafte Superabsorber sowie ein Verbund beinhaltend diese Superabsorber hergestellt werden können. Insbesondere sollte sich durch dieses Verfahren die Retention und das Absorptionsvermögen der Superabsorber unter einer Druckbelastung unabhängig voneinander verbessern lassen können.

Auch lag der vorliegenden Erfindung die Aufgabe zugrunde, Hygieneartikel mit einem hohen Gehalt an wasserabsorbierenden Polymergebilden bereitzustellen, welche durch eine besonders gute Hautverträglichkeit und weiterhin guten Absorptionseigenschaften, insbesondere einem guten Aufnahmevermögen für Körperflüssigkeiten, gekennzeichnet sind.

Einen Beitrag zur Lösung der vorstehend genannten Aufgaben liefert ein wasserabsorbierendes Polymergebilde, welches zu mindestens 50 Gew.-% auf carboxylatgruppenhaltigen Monomeren basiert, dessen Oberfläche mit einer Kombination aus einem wasserlöslichen Salz des Aluminiums und einem pulverförmigen Zinkoxidin Kontakt gebracht worden ist.

Völlig überraschend, jedoch nicht minder vorteilhaft wurde festgestellt, dass sich die Retentionseigenschaften von Superabsorberpartikeln beispielsweise durch das Behandeln der Oberflächen wasserabsorbierender Polymergebilde, welche zu mindestens 50 Gew.-% auf carboxylatgruppenhaltigen Monomeren basieren, mit einer Kombination aus einem wasserlöslichen Salz des Aluminiums und einem pulverförmigen Zinkoxidverbessern lassen. Insbesondere wurde beobachtet, dass durch die Oberflächenbehandlung der wasserabsorbierenden Polymergebilde mit der Kombination aus einem wasserlöslichen Salz des Aluminiums und dem pulverförmigen Zinkoxiddie bei der Oberflächennachvernetzung beobachtete Verminderung der Retention deutlich schwächer ausgeprägt ist als bei oberflächennachvernetzten Polymergebilden, deren Oberfläche nicht mit dieser Kombination aus wasserlöslichem Salz des Aluminiums und pulverförmigem Zinkoxidin Kontakt gebracht worden ist. Durch die erfindungsgemäße Oberflächenmodifizierung ist es beispielsweise möglich, Polymergebilde mit einem hohen Grad an Primärvernetzung, welche durch einen entsprechende geringen Gehalt an löslichen Anteilen und ein entsprechend geringes Retentionsvermögen gekennzeichnet sind, durch eine nach der Polymerisation erfolgende Oberflächenbehandlung in ihrem Retentionsvermögen zu verbessern, so dass Polymergebilde mit einem ausgezeichneten R/LA-Verhältnis erhalten werden können.

Erfindungsgemäß bevorzugte Polymergebilde sind Fasern, Schäume oder Teilchen, wobei Fasern und Teilchen bevorzugt und Teilchen besonders bevorzugt sind.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 im Bereich von 10 bis 3000 µm, vorzugsweise 20 bis 2000 µm und besonders bevorzugt 150 bis 850 µm oder 150 bis 600 µm aufweisen. Dabei ist es insbesondere bevorzugt, dass der Anteil der Polymerteilchen mit einer Partikelgröße in einem Bereich von 300 bis 600 µm mindestens 30 Gew.-%, besonders bevorzugt mindestens 40 Gew.-% und am meisten bevorzugt mindestens 50 Gew.-%, bezogen auf das Gesamtgewicht der nachvernetzten, wasserabsorbierenden Polymerteilchen, beträgt.

In einer bevorzugten Ausführungsform der erfindungsgemäßen wasserabsorbierenden Polymergebilde, welche zu mindestens 50 Gew.-% auf carboxylatgruppenhaltigen Monomeren basieren, basieren diese auf
(α1) 20-99,999 Gew.-%, bevorzugt 55-98,99 Gew.-% und besonders bevorzugt 70-98,79 Gew.-% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
(α2) 0-80 Gew.-%, vorzugsweise 0-44,99 Gew.-% und besonders bevorzugt 0,1-44,89 Gew.-% polymerisierten, monoethylenisch ungesättigten, mit (α1) copolymerisierbaren Monomeren,
(α3) 0,001-5 Gew.-%, vorzugsweise 0,01-3 Gew.-% und besonders bevorzugt 0,01-2,5 Gew.-% eines oder mehrerer Vernetzer,
(α4) 0,001 bis 5 Gew.-%, vorzugsweise 0,01 bis 2,5 Gew.-% und am meisten bevorzugt 0,1 bis 1 Gew.-% des wasserlöslichen Salzes des Aluminiums,
(α5) 0,001-5 Gew.-%, vorzugsweise 0,01-2,5 Gew.-% und am meisten bevorzugt 0,1-1 Gew.-% des pulverförmigen Zinkoxids,
(α6) 0-30 Gew.-%, vorzugsweise 0-5 Gew.-% und besonders bevorzugt 0,1-5 Gew.-% eines wasserlöslichen Polymeren,
(α7) 0-20 Gew.-%, vorzugsweise 2,5-15 Gew.-% und besonders bevorzugt 5-10 Gew.-% Wasser, sowie
(α8) 0-20 Gew.-%, vorzugsweise 0-10 Gew.-% und besonders bevorzugt 0,1-8 Gew.-% eines oder mehrerer Hilfsmittel, wobei die Summe der Gewichtsmengen (α1) bis (α8) 100 Gew.-% beträgt.

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen, deren Offenbarung hiermit als Referenz eingeführt wird. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen.

Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als "Mixed-Bed Ion-Exchange Absorbent Polymers" (MBIEA-Polymere) bezeichnet und sind unter anderem in der WO 99/34843 A1 offenbart. Die Offenbarung der WO 99/34843 A1 wird hiermit als Referenz eingeführt und gilt somit als Teil der Offenbarung. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte ethylenisch ungesättigte, säuregruppenhaltige Monomere (α1) sind vorzugsweise diejenigen Verbindungen, die in der WO 2004/037903 A2, die hiermit als Referenz eingeführt wird und somit als Teil der Offenbarung gilt, als ethylenisch ungesättigte, säuregruppenhaltige Monomere (α1) genannt werden.

Besonders bevorzugte ethylenisch ungesättigte, säuregruppenhaltige Monomere (α1) sind Acrylsäure und Methacrylsäure, wobei Acrylsäure am meisten bevorzugt ist.

Gemäß einer Ausführungsform des erfindungsgemäßen Verfahrens werden wasserabsorbierende Polymergebilde, welche zu mindestens 50 Gew.-% auf carboxylatgruppenhaltigen Monomeren basieren, eingesetzt, bei denen die monoethylenisch ungesättigten, mit (α1) copolymerisierbare Monomere (α2) Acrylamide, Methacrylamide oder Vinylamide sind.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth)acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Gemäß einer anderen Ausführungsform des erfindungsgemäßen Verfahrens werden wasserabsorbierende Polymergebilde, welche zu mindestens 50 Gew.-% auf carboxylatgruppenhaltigen Monomeren basieren, eingesetzt, bei denen die monoethylenisch ungesättigten, mit (α1) copolymerisierbare Monomere (α2) wasserlösliche Monomere sind. In diesem Zusammenhang sind insbesondere Alkoxypolyalkylenoxid(meth)acrylate wie Methoxypolyethylenglykol(meth)acrylate bevorzugt.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)-acrylat bevorzugt.

Die monoethylenisch ungesättigten, mit (α1) copolymerisierbaren Monomere (α2) umfassen weiterhin Methylpolyethylenglykol-allylether, Vinylacetat, Styrol und Isobutylen.

Als Vernetzer (α3) werden vorzugsweise diejenigen Verbindungen eingesetzt, die in der WO 2004/037903 A2 als Vernetzer (α3) genannt werden. Unter diesen Vernetzern sind wasserlösliche Vernetzer besonders bevorzugt. Am meisten bevorzugt sind dabei N,N'-Methylenbisacrylamid, Polyethylenglykoldi-(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als Metallsalz (α4) können alle dem Fachmann bekannten wasserlöslichen Salze des Aluminiums eingesetzt werden. Unter "wasserlöslich" werden dabei Metallsalze verstanden, von denen bei einer Temperatur von 25°C mindestens 1 g, besonders bevorzugt mindestens 10 g, darüber hinaus bevorzugt mindestens 100 g und am meisten bevorzugt mindestens 500 g in einem Liter destilliertem Wasser löslich sind.

Unter den vorstehend genannten, wasserlöslichen Salzen des Aluminiumssind insbesondere Sulfate, Sulfite, Sulfide, Chloride, Bromide, Iodide, Nitrate, Nitrite, Phosphate, Phosphite, Carbonate, Hydrogencarbonate, Hydroxide, Acetate, Lactate und Oxalate bevorzugt, wobei Sulfate am meisten bevorzugt sind.

Weiterhin ist es erfindungsgemäß , dass das Metallkation des Metallsalzes Al³⁺ ist.

Folgende wasserlöslichen Salze des Aluminiums sind in den erfindungsgemäßen wasserabsorbierenden Polymergebilden vorzugsweise als Oberflächenbehandlungsmittel enthalten: Aluminiumchlorid, Polyaluminiumchlorid, Aluminiumsulfat, Aluminiumnitrat, bis-Aluminium-Kalium-Sulfat, bis-Aluminium-NatriumSulfat, Aluminiumlactat, Aluminiumoxalat, Aluminiumcitrat, Aluminiumglyoxylat, Aluminiumsuccinat, Aluminiumitaconat, Aluminiumcrotonat, Aluminiumbutyrat, Aluminiumsorbat, Aluminiummalonat, Aluminiumbenzoat, Aluminiumtartrat, Aluminiumpyruvat, Aluiniumvalerat, Aluminiumformat, Aluminiumglutarat, Aluminiumpropanat oder Aluminiumacetat, wobei AlCl₃ × 6H₂0, NaAl(S0₄)₂ × 12H₂O, Al(NO₃)₃ × 9H₂O, KAl(S0₄)₂ × 12H₂0 oder Al₂(SO₄)₃ × 14-18H₂O sowie die entsprechenden wasserfreien Salze. Eine weitere bevorzugte Verbindung, die als Salz aufgefasst wird, ist Al(O)OH.

Als Oxid eines Metals wird pulverförmiges Zinkoxid eingesetzt. Dabei ist es besonders bevorzugt, dass das mindestens 50 Gew.-%, vorzugsweise mindestens 75 Gew.-% und am meisten bevorzugt mindestens 90 Gew.-% des pulverförmigen Zinkoxidseine durch Siebanalyse (für Partikelgrößen größer als 10 µm) oder Laserdiffraktometrie (für Partikelgrößen kleiner als 10 µm) bestimmte Partikelgröße in einem Bereich von 10 bis 1.000.000 nm, besonders bevorzugt in einem Bereich von 12 bis 500.000 nm und am meisten bevorzugt in einem Bereich von 15 bis 5000 nm aufweisen. Weiterhin ist es bevorzugt, dass das pulverförmige Zinkoxid ein Gewichtsmittel der Partikelgröße in einem Bereich von 15 bis 5000 nm, vorzugsweise in einem Bereich von 20 bis 3000 nm und am meisten bevorzugt in einem Bereich von 100 bis 2000 nm aufweist.

Die erfindungsgemäßen wasserabsorbierenden Polymergebilde, welche zu mindestens 50 Gew.-% auf carboxylatgruppenhaltigen Monomeren basieren, beinhalten als Oxid eines Metalls einpulverförmiges Zinkoxid.

In einer besonderen Ausführungsform der erfindungsgemäßen wasserabsorbierenden Polymergebilde, welche zu mindestens 50 Gew.-% auf carboxylatgruppenhaltigen Monomeren basieren, beinhalten diese als pulverförmiges Zinkoxidein feinteiliges Zinkoxid, welche zu mindestens 50 Gew.-% auf gegebenenfalls agglomerierten Partikeln mit einer Partikelgröße in einem Bereich von etwa 10 nm bis 500 µm basiert. Derartige feinteilige Zinkoxide sind beispielsweise unter der Handelbezeichnung "Nanox^{®}" von Elementis Specialities, USA, erhältlich.

Erfindungsgemäße wasserabsorbierende Polymergebilde, welche zu mindestens 50 Gew.-% auf carboxylatgruppenhaltigen Monomeren basieren, sind Polymergebilde, deren Oberfläche mit einem wasserlöslichen Salz des Aluminiums als Metallsalz und mit einem pulverförmigen Zinkoxid als Oxid eines Metalls in Kontakt gebracht worden sind.

Als wasserlösliche Polymere (α6) können in den Polymergebilden wasserlösliche Polymere, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsmittel (α8) sind vorzugsweise Stellmittel, Geruchsbinder, oberflächenaktive Mittel oder Antioxidatien sowie diejenigen Additive, die zur Herstellung der Polymergebilde eingesetzt wurden (Initiatoren usw.) in den Polymergebilden enthalten.

In einer besonderen Ausführungsform der erfindungsgemäßen Polymergebilde basieren diese zu mindestens 50 Gew.-%, vorzugsweise zu mindestens 70 Gew.-% und darüber hinaus bevorzugt zu mindestens 90 Gew.-% auf carboxylatgruppentragenden Monomeren. Es ist erfindungsgemäß weiterhin bevorzugt, dass die Komponente (α1) zu mindestens 50 Gew.-%, vorzugsweise zu mindestens 70 Gew.-% aus Acrylsäure besteht, die vorzugsweise zu mindestens 20 Mol-%, besonders bevorzugt zu mindestens 50 Mol-% und darüber hinaus bevorzugt in einem Bereich von 60 bis 85 Mol-% neutralisiert ist.

In einer anderen bevorzugten Ausführungsform der erfindungsgemäßen Polymergebilde weisen diese eine Kern-Schale-Struktur auf und umfassen einen Innenbereich (Kern) und einen den Innenbereich umgebenden Aussenbereich (Schale) auf, wobei das Polymergebilde im Aussenbereich stärker vernetzt ist als im Innenbereich.

Weiterhin sind die erfindungsgemäßen wasserabsorbierenden Polymergebilde, deren Oberfläche mit einem wasserlöslichen Salz des Aluminiumsund einem pulverförmigen Zinkoxidin Kontakt gebracht worden sind, durch ein R/LA-Verhältnis von mindestens 2,00 g/g, besonders bevorzugt von mindestens 2,59 g/g·%, darüber hinaus bevorzugt von mindestens 2,8 g/g·% und am meisten bevorzugt mindestens 3 g/g·% gekennzeichnet. Dabei ist R der gemäß ERT 441.2-02 (ERT = EDANA Recommended Test Method) bestimmte CRC-Wert (CRC = Centrifugation Retention Capacity; im Falle von Partikeln jeweils bestimmt für die gesamte Partikelfraktion) und LA der gemäß ERT 470.2-02 bestimmte Gehalt an Extractables (löslichen Anteilen).

Weiterhin sind die erfindungsgemäßen wasserabsorbierenden Polymergebilde, welche zu mindestens 50 Gew.-% auf carboxylatgruppenhaltigen Monomeren basieren, vorzugsweise durch ein nach ERT 442.2-02 bestimmte Absorption unter einem Druck von 50 g/cm² von mindestens 15 g/g, vorzugsweise mindestens 17 g/g und am meisten bevorzugt mindestens 19 g/g gekennzeichnet (im Falle von Partikeln jeweils bestimmt für die gesamte Partikelfraktion).

Einen Beitrag zur Lösung der eingangs genannten Aufgaben liefert auch ein Verfahren zur Behandlung der Oberfläche wasserabsorbierender Polymergebilde, umfassend die Verfahrensschritte
i) Bereitstellen eines unbehandelten, wasserabsorbierenden Polymergebildes, welches zu mindestens 50 Gew.-% auf carboxylatgruppenhaltigen Monomeren basiert;
ii) in Kontakt bringen der Oberfläche des unbehandelten, wasserabsorbierenden Polymergebildes mit einer Kombination aus einem wasserlöslichen Salz des Aluminiumsund einem pulverförmigen Zinkoxidbei einer Temperatur in einem Bereich von 30 bis 300°C, vorzugsweise in einem Bereich von 125 bis 250°C und am meisten bevorzugt in einem Bereich von 150 bis 200°C.

"Unbehandelt" im Sinne der vorliegenden Erfindung bedeutet, dass die wasserabsorbierenden Polymergebilde noch nicht mit der Kombination aus einem Metallsalz und einem Oxid eines Metalls in Kontakt gebracht worden sind. Die Bezeichnung "unbehandelt" schließt hingegen nicht aus, dass die wasserabsorbierenden Polymergebilde mittels anderer Oberflächenmodifizierungsmaßnahmen, wie etwa der Oberflächennachvernetzung, modifiziert sein können. Dabei ist es erfindungsgemäß besonders bevorzugt, dass das unbehandelte wasserabsorbierende Polymergebilde einen gemäß ERT 441.2-02 bestimmten CRC-Wert von mindestens 30 g/g, besonders bevorzugt mindestens 35 glg, mehr bevorzugt mindestens 36 g/g, noch mehr bevorzugt mindestens 38 g/g und am meisten bevorzugt mindestens 40 g/g aufweist, wobei vorzugsweise ein CRC-Wert von 75 g/g, besonders bevorzugt 70 g/g, darüber hinaus bevorzugt 65 g/g, darüber hinaus noch mehr bevorzugt 60 g/g und am meisten bevorzugt 55 g/g nicht überschritten wird (im Falle von Partikeln jeweils bestimmt für die gesamte Partikelfraktion). Weiterhin umfasst der Begriff "unbehandeltes wasserabsorbierendes Polymergebilde" auch Polymergebilde, die noch nicht vollständig getrocknet sind und noch einen Wassergehalt von mehr als 10 Gew.-%, vorzugsweise mehr als 20 Gew.-% und am meisten bevorzugt mehr als 30 Gew.-%, jeweils bezogen auf das Gesamtgewicht des unbehandelten, wasserabsorbierenden Polymergebildes, aufweisen, wobei vorzugsweise ein Wassergehalt von 75 Gew.-%, bevorzugt von 50 Gew.-% nicht überschritten wird.

Als unbehandelte, wasserabsorbierende Polymergebilde, welche zu mindestens 50 Gew.-% auf carboxylatgruppenhaltigen Monomeren basieren, werden dabei in dem erfindungsgemäßen Verfahren vorzugsweise Polymere eingesetzt, welche erhalten wurden durch ein Verfahren umfassend die Verfahrensschritte:
a) radikalische Polymerisation säuregruppen-tragender, ethylenisch ungesättigter, gegebenenfalls teilneutralisierter Monomere in Gegenwart eines Vernetzers unter Bildung eines Hydrogels;
b) gegebenenfalls Zerkleinern des Hydrogels;
c) Trocknen des gegebenenfalls zerkleinerten Hydrogels unter Erhalt wasserabsorbierender Polymergebilde;
d) gegebenenfalls Zermahlen des so erhaltenen absorbierenden Polymergebildes und absieben auf eine gewünschte Partikelgrößenfraktion;
e) gegebenenfalls weitere Oberflächenmodifizierungen der so erhalten wasserabsorbierenden Polymergebilde;

Das auf diese Art und Weise erhaltene, definitionsgemäß "unbehandelte" Polymergebilde wird anschließend mit der Kombination aus einem wasserlöslichen Salz des Aluminiums und einem pulverförmigen Zinkoxid in Kontakt gebracht, wobei es bevorzugt ist, dass das wasserlösliche Salz des Aluminiums und das pulverförmige Zinkoxidin Form eines Fluids F₁ umfassend ein Lösungsmittel, das wasserlösliche Salz des Aluminiumsund das pulverförmige Zinkoxid mit dem wasserabsorbierenden Polymergebilde in Kontakt gebracht werden. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass das wasserlösliche Salz des Aluminiums in dem Fluid F₁ in einer Menge in einem Bereich von 0,1 bis 50 Gew.-%, besonders bevorzugt 1 bis 40 Gew.-% und am meisten bevorzugt 5 bis 25 Gew.-% und das pulverförmige Zinkoxidin dem Fluid F₁ in einer Menge in einem Bereich von 0,1 bis 50 Gew.-%, besonders bevorzugt 1 bis 40 Gew.-% und am meisten bevorzugt 5 bis 25 Gew.-%, jeweils bezogen auf das Gesamtgewicht des Lösungsmittels , enthalten ist. Denkbar ist auch, das wasserlösliche Salz des Aluminiums und das pulverförmigen Zinkoxid in getrennten Fluiden einzusetzen.

Weiterhin ist es erfindungsgemäß bevorzugt, dass das wasserlösliche Salz des Aluminiums in einer Menge in einem Bereich von 0,001 bis 5 Gew.-%, vorzugsweise 0,01 bis 2,5 Gew.-% und noch mehr bevorzugt 0,1 bis 1 Gew.-% und das pulverförmigen Zinkoxid in einer Menge in einem Bereich von 0,001 bis 5 Gew.-%, vorzugsweise 0,01 bis 2,5 Gew.-% und noch mehr bevorzugt 0,1 bis 1 Gew.-%, jeweils bezogen auf das Gewicht des unbehandelten wasserabsorbierenden Polymergebildes, mit dem unbehandelten wasserabsorbierenden Polymergebilde in Kontakt gebracht werden.

Geeignete Mischaggregate zum Aufbringen des Fluids F₁ sind wiederum der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Die im Verfahrensschritt a) erfolgende radikalische Polymerisation erfolgt vorzugsweise in wässriger Lösung, wobei diese wässrige Lösung neben Wasser als Lösungsmittel vorzugsweise
(α1) die ethylenisch ungesättigten, säuregruppen-tragenden Monomeren oder deren Salze, wobei Acrylsäure als säuregruppentragendes Monomer besonders bevorzugt ist,
(α2) gegebenenfalls monoethylenisch ungesättigte, mit (α1) copolymerisierbare Monomere,
(α3) den Vernetzer,
(α6) gegebenenfalls ein wasserlösliches Polymer, sowie
(α8) gegebenenfalls einen oder mehrere Hilfsmittel

### beinhaltet.

Als ethylenisch ungesättigte, säuregruppen-tragenden Monomere (α1), als monoethylenisch ungesättigte, mit (α1) copolymerisierbare Monomere (α2), als Vernetzer (α3), als wasserlösliche Polymere (α6) und als Hilfsmittel (α8) sind wiederum diejenigen Verbindungen bevorzugt, die bereits eingangs im Zusammenhang mit den erfindungsgemäßen Polymergebilden als ethylenisch ungesättigte, säuregruppen-tragenden Monomere (α1), als monoethylenisch ungesättigte, mit (α1) copolymerisierbare Monomere (α2), als Vernetzer (α3), als wasserlösliche Polymere (α6) und als Hilfsmittel (α8) genannt wurden.

Aus den vorgenannten Monomeren, Comonomeren, Vernetzern, wasserlöslichen Polymeren und Hilfsstoffen lassen sich die wasserabsorbierenden Polymergebilde, welche zu mindestens 50 Gew.-% auf carboxylatgruppenhaltigen Monomeren basieren, durch verschiedene Polymerisationsweisen herstellen. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818. Die Offenbarungen werden hiermit als Referenz eingeführt und gelten somit als Teil der Offenbarung.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde, welche zu mindestens 50 Gew.-% auf carboxylatgruppenhaltigen Monomeren basieren, ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der Polymergebilde, welche zu mindestens 50 Gew.-% auf carboxylatgruppenhaltigen Monomeren basieren, angewendet werden. Gemäß diesen Prozessen wird eine wässrige, teilneutralisierte Lösung der Monomeren (α1), und (α2), gegebenenfalls beinhaltend wasserlösliche Polymere und Hilfsstoffe, mit Hilfe von Schutzkolloiden und/oder Emulgatoren in einem hydrophoben, organischen Lösungsmittel dispergiert und durch Radikalinitiatoren die Polymerisation gestartet.

Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren (α6) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers und/oder durch Reaktion geeigneter Vernetzer mit funktionellen Gruppen des Polymeren während der Polymerisationsschritte erfolgen. Die Verfahren sind beispielsweise in den Veröffentlichungen US 4,340,706, DE 37 13 601, DE 28 40 010 und WO 96/05234 A1 beschrieben, deren entsprechende Offenbarung hiermit als Referenz eingeführt wird.

Die bei der Lösungspolymerisation oder der inversen Suspensions- und Emulsionspolymerisation im Verfahrensschritt a) erhaltenen Hydrogele werden im Verfahrensschritt c) getrocknet.

Insbesondere im Falle der Lösungspolymerisation ist es jedoch bevorzugt, dass die Hydrogele vor der Trocknung zunächst in einem zusätzlichen Verfahrensschritt b) zerkleinert werden. Dieses Zerkleinern erfolgt durch dem Fachmann bekannte Zerkleinerungsvorrichtungen, wie etwa einem Fleischwolf.

Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Hydrogels im Verfahrensschritt c) bis zu einem Wassergehalt von 0,5 bis 25 Gew.-%, vorzugsweise von 1 bis 10 Gew.-% erfolgt, wobei die Trocknungstemperaturen üblicherweise in einem Bereich von 100 bis 200°C liegen.

Die im Verfahrensschritt c) erhaltenen, getrockneten wasserabsorbierenden Polymergebilde, welche zu mindestens 50 Gew.-% auf carboxylatgruppenhaltigen Monomeren basieren, können, insbesondere dann, wenn sie durch Lösungspolymerisation erhalten wurden, in einem weiteren Verfahrensschritt d) noch zermahlen und auf die eingangs genannte Wunschkorngröße abgesiebt werden. Das Zermahlen der getrockneten, wasserabsorbierenden Polymergebilde erfolgt vorzugsweise in geeigneten, mechanischen Zerkleinerungsvorrichtungen, wie etwa einer Kugelmühle.

Im Anschluss an die Trocknung der Hydrogele und der gegebenenfalls erfolgten weiteren Konfektionierung der getrockneten wasserabsorbierenden Polymergebilde, welche zu mindestens 50 Gew.-% auf carboxylatgruppenhaltigen Monomeren basieren, können diese in einem weiteren Verfahrenschritt e) im Oberflachenbereich modifiziert werden (neben der im nachfolgenden noch eingehender beschriebenen Modifizierung mit der Kombination aus dem wasserlöslichen Salz des Aluminiums und dem pulverförmigen Zinkoxid).

Als bevorzugte Modifizierungsmaßnahme sei hier die Oberflächennachvernetzung genannt, bei der die getrockneten Polymergebilde, welche zu mindestens 50 Gew.-% auf carboxylatgruppenhaltigen Monomeren basieren, oder aber das noch nicht getrocknete, jedoch vorzugsweise bereits zerkleinerte Hydrogel mit einem vorzugsweise organischen, chemischen Oberflächennachvernetzer in Kontakt gebracht wird. Dabei wird der Nachvernetzer insbesondere dann, wenn er unter den Nachvernetzungsbedingungen nicht flüssig ist, vorzugsweise in Form eines Fluids F₂ umfassend den Nachvernetzer sowie ein Lösungsmittel mit den Polymerteilchen bzw. dem Hydrogel in Kontakt gebracht. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvernetzer in dem Fluid F₂ in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Fluids F₂, enthalten ist.

Das in Kontakt bringen des Polymergebildes, welches zu mindestens 50 Gew.-% auf carboxylatgruppenhaltigen Monomeren basiert, bzw. des zerkleinerten Hydrogels mit dem Fluid F₂ beinhaltend den Nachvernetzer erfolgt im erfindungsgemäßen Verfahren vorzugsweise durch gutes Vermischen des Fluids F₂ mit dem Polymergebilde.

Geeignete Mischaggregate zum Aufbringen des Fluids F₂ sind z. B. der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Das Polymergebilde, welches zu mindestens 50 Gew.-% auf carboxylatgruppenhaltigen Monomeren basieren, wird in dem erfindungsgemäßen Verfahren bei der Nachvernetzung vorzugsweise mit höchstens 20 Gew. -%, besonders bevorzugt mit höchstens 15 Gew.-%, darüber hinaus bevorzugt mit höchstens 10 Gew. -%, darüber hinaus noch mehr bevorzugt mit höchstens 5 Gew.-% an Lösungsmittel, vorzugsweise Wasser, in Kontakt gebracht.

Bei Polymergebilden, welche zu mindestens 50 Gew.-% auf carboxylatgruppenhaltigen Monomeren basieren, in der Form von vorzugsweise kugelförmigen Teilchen ist es erfindungsgemäß weiterhin bevorzugt, dass das in Kontakt bringen derart erfolgt, dass lediglich der Aussenbereich, nicht jedoch der innere Bereich der teilchenförmigen Polymergebilde mit dem Fluid F₂ und somit dem Nachvernetzer in Kontakt gebracht werden.

Als Nachvernetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvernetzer), in einer Additionsreaktion oder in einer Ringöffnungsreaktion reagieren können. Als Nachvernetzer sind im erfindungsgemäßen Verfahren diejenigen bevorzugt, die in WO 2004/037903 A2 als Vernetzer der Vernetzerklassen II genannt wurden.

Unter diesen Verbindungen sind als Nachvernetzer besonders bevorzugt Kondensationsvemetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1, 3-dioxolan-2-on, 1,3-Dioxan-2-on, 4-Methyl-1,3-dioxan-2-on, 4,6-Dimethyl-1,3-dioxan-2-on sowie 1,3-Dioxolan-2-on.

Nachdem die Polymergebilde bzw. die Hydrogele mit dem Nachvernetzer bzw. mit dem Fluid beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Aussenbereich der Polymergebilde im Vergleich zum Innenbereich stärker vernetzt wird (=Nachvernetzung). Die Zeitdauer der Wärmebehandlung wird durch die Gefahr, dass das gewünschte Eigenschaftsprofil der Polymergebilde infolge von Hitzeeinwirkung zerstört wird, begrenzt.

Die vorstehend beschriebene Oberflächenmodifizierung mittels Nachvernetzung kann auch Zeitgleich mit der Modifizierung der wasserabsorbierenden Polymergebilde, welches zu mindestens 50 Gew.-% auf carboxylatgruppenhaltigen Monomeren basiert, mit der Kombination aus einem wasserlöslichen Salz des Aluminiumsund einem pulverförmigen Zinkoxiderfolgen. In diesem Fall ist es bevorzugt, den Nachvernetzer bereits dem Fluid F₁ zuzusetzen oder das Fluid F₁ und das Fluid F₂ zeitgleich mit dem wasserabsorbierenden Polymergebilde in Kontakt zu bringen.

In einer Ausführungsform des erfindungsgemäßen Verfahrens erfolgt das in Kontakt bringen der unbehandelten wasserabsorbierenden Polymergebilde , welche zu mindestens 50 Gew.-% auf carboxylatgruppenhaltigen Monomeren basieren, mit der Kombination aus dem wasserlöslichen Salz des Aluminiums und dem pulverförmigen Zinkoxid nachdem die wasserabsorbierenden Polymergebilde mit einem in WO 2004/037903 A2 als Vernetzer der Vernetzerklassen II genannten Nachvernetzer nachvernetzt worden sind.

In einer anderen Ausführungsform des erfindungsgemäßen Verfahrens erfolgt das in Kontakt bringen der unbehandelten wasserabsorbierenden Polymergebilde , welche zu mindestens 50 Gew.-% auf carboxylatgruppenhaltigen Monomeren basieren, mit der Kombination aus dem wasserlöslichen Salz des Aluminiums und dem pulverförmigen Zinkoxid zeitgleich mit dem in Kontakt bringen der wasserabsorbierenden Polymergebilde mit einem in WO 2004/037903 A2 als Vernetzer der Vernetzerklassen II genannten Nachvernetzer, wobei es in diesem Fall bevorzugt ist, den Vernetzer der Vernetzerklasse II dem Fluid F₁ zuzusetzen. In diesem Fall ist es bevorzugt, die mit dem Fluid F₁ umfassend das wasserlösliche Salz des Aluminiums, das pulverförmigen Zinkoxid und den Vernetzer der Vernetzerklasse II in Kontakt gebrachten unbehandelten wasserabsorbierenden Polymergebilde auf eine Temperatur in einem Bereich von 50 bis 300 °C, vorzugsweise 75 bis 275°C und am meisten bevorzugt 150 bis 250°C zu erhitzen.

In einer weiteren Ausführungsform des erfindungsgemäßen Verfahrens erfolgt das in Kontakt bringen der unbehandelten wasserabsorbierenden Polymergebilde, welche zu mindestens 50 Gew.-% auf carboxylatgruppenhaltigen Monomeren basieren, mit der Kombination aus dem wasserlöslichen Salz des Aluminiums und dem pulverförmigen Zinkoxid bevor das wasserabsorbierende Polymergebilde mit einem in WO 2004/037903 A2 als Vernetzer der Vernetzerklassen II genannten Nachvernetzer in Kontakt gebracht worden ist.

Gemäß einer besonderen Ausführungsform des erfindungsgemäßen Verfahrens zur Behandlung der Oberfläche wasserabsorbierender Polymergebilde, welche zu mindestens 50 Gew.-% auf carboxylatgruppenhaltigen Monomeren basieren, wird im Verfahrensschritt ii) die Oberfläche des unbehandelten wasserabsorbierenden Polymergebildes mit dem wasserlöslichen Salz des Aluminiums und dem pulverförmigen Zinkoxid in Kontakt gebracht, wobei sowohl das wasserlösliche Salz des Aluminiums als auch das Zinkoxid jeweils in Pulverform vorliegen.

Bei dieser besonderen Ausführungsform des erfindungsgemäßen Verfahrens zur Behandlung der Oberfläche wasserabsorbierender Polymergebilde, welche zu mindestens 50 Gew.-% auf carboxylatgruppenhaltigen Monomeren basieren, umfasst das Verfahren vorzugsweise die folgenden Verfahrensschritte:
i) Bereitstellen des unbehandelten, vorzugsweise jedoch bereits oberflächennachvernetzten wasserabsorbierenden Polymergebildes, welches zu mindestens 50 Gew.-% auf carboxylatgruppenhaltigen Monomeren basiert;
ii) in Kontakt bringen der Oberfläche des unbehandelten, vorzugsweise jedoch bereits oberflächennachvernetzten wasserabsorbierenden Polymergebildes mit einer Feinstteilchenkomponente umfassend eine Kombination aus einem wasserlöslichen Salz des Aluminiums und einem pulverförmigen Zinkoxidbei einer Temperatur in einem Bereich von 30 bis 300°C, besonders bevorzugt von 100 bis 300°C, darüber hinaus bevorzugt in einem Bereich von 125 bis 250°C und am meisten bevorzugt in einem Bereich von 150 bis 200°C.

In diesem Zusammenhang ist es insbesondere bevorzugt, dass mindestens 50 Gew.-%, besonders bevorzugt mindestens 75 Gew.-%, darüber hinaus noch mehr bevorzugt mindestens 95 Gew.-% und am meisten bevorzugt mindestens 99 Gew.-% des in Pulverform vorliegenden wasserlöslichen Salz des Aluminiums einen mittleren Teilchendurchmesser (Gewichtsmittel) in einem Bereich von 10 bis 1.000 µm, vorzugsweise von 50 µm bis 800 µm, besonders bevorzugt von 100 bis 600 µm und am meisten bevorzugt von 200 bis 400 µm aufweisen, jeweils bestimmt durch dem Fachmann bekannte Verfahren zur Partikelgrößenbestimmung, beispielsweise durch Siebanalyse oder mittels eines Coulter-Counters. Weiterhin ist es bevorzugt, dass mindestens 50 Gew.-%, vorzugsweise mindestens 75 Gew.-% und am meisten bevorzugt mindestens 90 Gew.-% des pulverförmigen Zinkoxid eine durch Siebanalyse (für Partikelgrößen größer als 10 µm) oder Laserdiffraktometrie (für Partikelgrößen kleiner als 10 µm) bestimmte Partikelgröße in einem Bereich von 10 bis 1.000.000 nm, besonders bevorzugt in einem Bereich von 12 bis 500.000 nm und am meisten bevorzugt in einem Bereich von 15 bis 5000 nm aufweisen. Weiterhin ist es bevorzugt, dass das pulverförmige Zinkoxid ein Gewichtsmittel der Partikelgröße in einem Bereich von 15 bis 5000 nm, vorzugsweise in einem Bereich von 20 bis 3000 nm und am meisten bevorzugt in einem Bereich von 100 bis 2000 nm aufweist.

Weiterhin kann es bei dieser besonderen Ausführungsform des erfindungsgemäßen Verfahrens bevorzugt sein, wenn die Feinstteilchenkomponente neben dem pulverförmigen wasserlöslichen Salz des Aluminiums und dem pulverförmigen Zinkoxid zusätzlich einen Binder beinhaltet, wobei auch dieser Binder vorzugsweise in partikulärer Form vorliegt und insbesondere zu mindestens 50 Gew.-%, besonders bevorzugt zu mindestens 75 Gew.-%, darüber hinaus noch mehr bevorzugt zu mindestens 95 Gew.-% und am meisten bevorzugt zu mindestens 99 Gew.-% auf Partikel mit einem mittleren Teilchendurchmesser (Gewichtsmittel) in einem Bereich von 10 bis 1.000 µm, vorzugsweise von 50 µm bis 800 µm, besonders bevorzugt von 100 bis 600 µm und am meisten bevorzugt von 200 bis 400 µm basiert, jeweils bestimmt durch dem Fachmann bekannte Verfahren zur Partikelgrößenbestimmung, beispielsweise durch Siebanalyse oder mittels eines Coulter-Counters.

In diesem Zusammenhang ist es insbesondere bevorzugt, dass der Binder als eine Binderhauptkomponente eine organische Verbindung beinhaltet, wobei die organische Verbindung vorzugsweise bei 20°C ein Feststoff ist.

Besonders bevorzugt ist es, dass die organische Verbindung ein vorzugsweise lineares Polymer ist, vorzugsweise ein lineares Polymer ausgewählt aus der Gruppe umfassend Polyurethane, Polyester, Polyamide, Polyesteramide, Polyolefine, Polyvinylester, Polyether, Polystyrole, Polyimide, insbesondere Polyetherimide, Polyimine, Schwefelpolymere, insbesondere Polysulfon, Polyacetale, insbesondere Polyoxymethylene, Fluorkunststoffe, insbesondere Polyvinylidenfluorid, Styrol-Olefin-Copolymere, Polyacrylate, Ethylen-Vinylacetat-Copolymere oder Gemische aus zwei oder mehr der genannten Polymere, wobei unter diesen Polymeren Polykondensate und unter diesen Polyether besonders bevorzugt und lineare Polyether am meisten bevorzugt sind.

Besonders geeignete lineare Polyether umfassen Polyalkylenglykole, insbesondere Polyethylenglykole, Polypropylenglykole, Poly(ethylen/propylen)glykole mit statistischer oder blockartiger Anordnung der Ethylen- oder Propylen-Monomere oder Mischungen aus mindesten zwei dieser Polyalkylenglykole.

Weitere geeignete, vorzugsweise lineare Polymere sind diejenigen Polymere, die in der DE-A-103 34 286 als "thermoplastische Klebstoffe" genannt werden. Der Offenbarungsgehalt der DE-A-103 34 286 hinsichtlich thermoplastischer Klebstoffe wird hiermit als Referenz eingeführt und bildet einen Teil der Offenbarung der vorliegenden Erfindung.

Wenn neben dem wasserlöslichen Salz des Aluminiums und dem pulverförmigen Zinkoxid auch ein Binder eingesetzt wird, ist es insbesondere bevorzugt, dass das in Kontakt bringen der Oberfläche des unbehandelten, wasserabsorbierenden Polymergebildes, welches zu mindestens 50 Gew.-% auf carboxylatgruppenhaltigen Monomeren basiert, mit der Feinstteilchenkomponente bei einer Temperatur in einem Bereich von 30 bis 200°C, besonders bevorzugt von 50 bis 160°C und am meisten bevorzugt in einem Bereich von 70 bis 140°C erfolgt. Bei diesen Temperaturen kommt es insbesondere auch zu einer Immobilisierung der Feinstteilchen auf der Oberfläche des unbehandelten, wasserabsorbierenden Polymergebildes.

Die Menge an Bindemittel, sofern eingesetzt, liegt vorzugsweise in einem Bereich von 0,0001 bis 5 Gew.-% und besonders bevorzugt in einem Bereich von 0,001 bis 2 Gew.-%, jeweils bezogen auf das Gewicht der wasserabsorbierenden Polymergebilde. Das Gewichtsverhältnis zwischen Feinstteilchenkomponente und Bindemittel liegt vorzugsweise in einem Bereich von Feinstteilchenkomponente : Bindemittel von 20 : 1 bis 1 : 20, besonders bevorzugt von 10 : 1 bis 1 : 10 und am meisten bevorzugt von 10 : 1 bis 2 : 1.

Bei der vorstehend beschriebenen, besonderen Ausführungsform des erfindungsgemäßen Verfahrens, bei der ein pulverfömiges, wasserlösliches Salz des Aluminiums und ein pulverförmiges Zinkoxidzur Behandlung der Oberfläche wasserabsorbierender Polymergebilde, welche zu mindestens 50 Gew.-% auf carboxylatgruppenhaltigen Monomeren basieren, eingesetzt wird, umfasst das Verfahren neben der Bereitstellung des unbehandelten, wasserabsorbierenden Polymergebildes im Verfahrensschritt i) auch die Bereitstellung einer Feinstteilchenkomponente umfassend die Kombination aus pulverförmigem, wasserlöslichen Salz des Aluminiums und pulverförmigem Zinkoxid sowie aus pulverförmigem Binder. Hinsichtlich der Art und Weise des in Kontakt bringens der Feinstteilchenkomponente mit dem unbehandelten, wasserabsorbierenden Polymergebilde sind unterschiedliche Verfahrensführungen denkbar:
- Gemäß der Variante V_{A} wird zunächst im Verfahrensschritt ii) eine Mischung aus Feinstteilchenkomponente und unbehandelten, vorzugsweise jedoch bereits oberflächennachvernetzten wasserabsorbierenden Polymergebilden, welche zu mindestens 50 Gew.-% auf carboxylatgruppenhaltigen Monomeren basieren, hergestellt und diese anschließend Zwecks Immobilisierung der Feinstteilchen auf die vorstehend genannte Temperatur erhitzt, wobei das wasserabsorbierende Polymergebilde bereits oberflächennachvernetzt sein kann oder wobei das wasserabsorbierende Polymergebilde zwar schon mit dem Nachvernetzter in Kontakt gebracht worden ist, aber noch nicht auf eine für eine Oberflächennachvernetzung erforderliche Temperatur erhitzt worden ist.
- Gemäß der Variante V_{B} werden zunächst vor dem Verfahrensschritt ii) die unbehandelten, vorzugsweise jedoch bereits oberflächennachvernetzten wasserabsorbierenden Polymergebilde, welche zu mindestens 50 Gew.-% auf carboxylatgruppenhaltigen Monomeren basieren, auf die vorstehend beschriebene Temperatur erhitzt und anschließend im Verfahrensschritt ii) diese vorgewärmten wasserabsorbierenden Polymergebilde mit der nicht-vorgewärmten Feinstteilchenkomponente vermischt.
- Gemäß der Variante V_{C} werden zunächst vor dem Verfahrensschritt ii) die unbehandelten, vorzugsweise jedoch bereits oberflächennachvernetzten wasserabsorbierenden Polymergebilde, welche zu mindestens 50 Gew.-% auf carboxylatgruppenhaltigen Monomeren basieren, und die Feinstteilchenkomponente getrennt jeweils auf die vorstehend beschriebene Temperatur erwärmt und anschließend im Verfahrensschritt ii) die vorgewärmten wasserabsorbierenden Polymergebilde mit der ebenfalls vorgewärmten Feinstteilchenkomponente vermischt. Gemäß einer besonderen Ausgestaltung dieser Variante V_{C} ist es bevorzugt, die Feinstteilchenkomponente nach dem Erwärmen und vor dem Vermischen mit den vorgewärmten wasserabsorbierenden Polymergebilden zunächst abzukühlen, vorzugsweise auf eine Temperatur in einem Bereich von 10 bis 100°C, besonders bevorzugt 15 bis 75°C und am meisten bevorzugt 20 bis 60°C, danach gegebenenfalls zu zerkleinern, beispielsweise mittels eines Mörsers, und dann die abgekühlte und gegebenenfalls zerkleinerte Feinstteilchenkomponente mit den vorgewärmten wasserabsorbierenden Polymergebilden zu vermischen.
- Gemäß der Variante V_{D} wird zunächst vor dem Verfahrensschritt ii) die Feinstteilchenkomponente auf die vorstehend beschriebene Temperatur erwärmt und anschließend im Verfahrensschritt ii) die vorgewärmte Feinstteilchenkomponente mit den nicht-vorgewärmten, unbehandelten, vorzugsweise jedoch bereits oberflächennachvernetzten wasserabsorbierenden Polymergebilde, welche zu mindestens 50 Gew.-% auf carboxylatgruppenhaltigen Monomeren basieren, vermischt. Gemäß einer besonderen Ausgestaltung dieser Variante V_{D} ist es bevorzugt, die Feinstteilchenkomponente nach dem Erwärmen und vor dem Vermischen mit den nicht-vorgewärmten wasserabsorbierenden Polymergebilden zunächst abzukühlen, vorzugsweise auf eine Temperatur in einem Bereich von 10 bis 100°C, besonders bevorzugt 15 bis 75°C und am meisten bevorzugt 20 bis 60°C, danach gegebenenfalls zu zerkleinern, beispielsweise mittels eines Mörsers, und dann die abgekühlte und gegebenenfalls zerkleinerte Feinstteilchenkomponente mit den nicht-vorgewärmten wasserabsorbierenden Polymergebilden zu vermischen.

Weiterhin kann es im Zusammenhang mit der vorstehend beschriebenen, besonderen Ausführungsform des erfindungsgemäßen Verfahrens, bei der ein pulverfömiges, wasserlösliches Salz des Aluminiums und ein pulverförmiges Zinkoxid zur Behandlung der Oberfläche wasserabsorbierender Polymergebilde, welche zu mindestens 50 Gew.-% auf carboxylatgruppenhaltigen Monomeren basieren, eingesetzt wird, vorteilhaft sein, wenn sich an den Verfahrensschritt ii) noch ein weiterer Verfahrensschritt iii) anschließt, in dem die Mischung aus unbehandeltem, wasserabsorbierenden Polymergebilde und Feinstteilchenkomponente noch für eine Zeitraum in einem Bereich von 10 Minuten bis 5 Stunden, besonders bevorzugt von 30 Minuten bis 3 Stunden gemischt wird, um eine möglichst homogene Verteilung der Feinstteilchen bzw. der Feinstteilchenagglomerate und der absorbierenden Polymergebilde zu ermöglichen, wobei hierzu dem Fachmann bekannte Mischvorrichtungen eingesetzt werden können. In diesem weiteren Verfahrensschritt kann die Mischung aus unbehandeltem, wasserabsorbierenden Polymergebilde und Feinstteilchenkomponente mit der Temperatur, die sie nach dem Immobilisieren im Verfahrensschritt ii) aufweist, in den Mischer eingebracht werden, wobei die Mischung dann im Verlaufe des Mischens vorzugsweise stetig auf eine niedrigere Temperatur, vorzugsweise auf Raumtemperatur, abgekühlt werden kann.

Einen weiteren Beitrag zur Lösung der eingangs beschriebenen Aufgaben liefern die durch das erfindungsgemäße Verfahren erhältlichen oberflächenbehandelten wasserabsorbierenden Polymergebilde, welche zu mindestens 50 Gew.-% auf carboxylatgruppenhaltigen Monomeren basieren.

Dabei ist es bevorzugt, dass die eingangs beschriebenen erfindungsgemäßen wasserabsorbierenden Polymergebilde, welche zu mindestens 50 Gew.-% auf carboxylatgruppenhaltigen Monomeren basieren, die gleichen Eigenschaften aufweisen wie die durch das erfindungsgemäßen Verfahren erhältlichen oberflächenbehandelten wasserabsorbierenden Polymergebilde. Es ist auch erfindungsgemäß bevorzugt, dass diejenigen Werte, die im Zusammenhang mit dem erfindungsgemäßen Verfahren und den erfindungsgemäßen Polymergebilden als Untergrenzen von erfindungsgemäßen Merkmalen ohne Obergrenzen angegeben wurden, das 20-fache, vorzugsweise das 10-fache und besonders bevorzugt das 5-fache des am meisten bevorzugten Wertes der Untergrenze besitzen.

Einen weiteren Beitrag zur Lösung der eingangs beschriebenen Aufgaben liefert ein Verbund, beinhaltend die erfindungsgemäßen wasserabsorbierenden Polymergebilde, welche zu mindestens 50 Gew.-% auf carboxylatgruppenhaltigen Monomeren basieren, bzw. die durch das erfindungsgemäße Verfahren erhältlichen oberflächenbehandelten wasserabsorbierenden Polymergebilde, welche zu mindestens 50 Gew.-% auf carboxylatgruppenhaltigen Monomeren basieren, und ein Substrat. Es ist dabei bevorzugt, dass die erfindungsgemäßen Polymergebilde und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich umfasst, welcher das erfindungsgemäße wasserabsorbierende Polymergebilde, welche zu mindestens 50 Gew.-% auf carboxylatgruppenhaltigen Monomeren basieren, in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm³, vorzugsweise mindestens 0,1 cm³ und am meisten bevorzugt mindestens 0,5 cm³ aufweist.

In einer besonders bevorzugten Ausführungsform des erfindungsgemäßen Verbundes handelt es sich um einen flächenförmigen Verbund, wie er in der WO 02/056812 A1 als "absorbent material" beschrieben ist. Der Offenbarungsgehalt der WO 02/056812 A1, insbesondere hinsichtlich des genauen Aufbaus des Verbundes, des Flächengewichtes seiner Bestandteile sowie seiner Dicke wird hiermit als Referenz eingeführt und stellt einen Teil der Offenbarung der vorliegenden Erfindung dar.

Einen weiteren Beitrag zur Lösung der Eingangs genannten Aufgaben liefert ein Verfahren zur Herstellung eines Verbundes, wobei die erfindungsgemäßen wasserabsorbierenden Polymergebilde, welche zu mindestens 50 Gew.-% auf carboxylatgruppenhaltigen Monomeren basieren, bzw. die durch das erfindungsgemäße Verfahren erhältlichen oberflächenbehandelten wasserabsorbierenden Polymergebilde, welche zu mindestens 50 Gew.-% auf carboxylatgruppenhaltigen Monomeren basieren, und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Gemäß einer besonderen Ausgestaltung des erfindungsgemäßen Verfahrens zur Herstellung eines Verbundes umfasst dieses Verfahren die folgenden Verfahrensschritte:
I) Bereitstellen eines Substrates;
II) Bereitstellen eines unbehandelten, vorzugsweise jedoch bereits oberflächennachvernetzten, wasserabsorbierenden Polymergebildes, welches zu mindestens 50 Gew.-% auf carboxylatgruppenhaltigen Monomeren basiert;
III) Bereitstellen einer Feinstteilchenkomponente;
IV) In Kontakt bringen des Substrates mit dem wasserabsorbierenden Polymergebilde;
V) in Kontakt bringen des wasserabsorbierenden Polymergebildes mit der Feinstteilchenkomponente;
VI) Immobilisieren mindestens eines Teils der Feinstteilchen auf der Oberfläche der wasserabsorbierenden Polymergebilde,
wobei die Feinstteilchenkomponente mindestens eine Kombination aus einem wasserlöslichen Salz des Aluminiums und einem pulverförmigen Zinkoxid ist. Besonders bevorzugt ist eine Mischung aus pulverförmigem, wasserlöslichem Salz des Aluminiums, pulverförmigem Zinkoxid und pulverförmigem Binder.

Gemäß einer Variante dieser besonderen Ausgestaltung des erfindungsgemäßen Verfahrens zur Herstellung eines Verbundes werden zunächst das Substrat und das unbehandelte, vorzugsweise jedoch bereits oberflächennachvernetzte, wasserabsorbierende Polymergebilde, welches zu mindestens 50 Gew.-% auf carboxylatgruppenhaltigen Monomeren basiert, miteinander in Kontakt gebracht, vorzugsweise dadurch, dass zunächst das Substrat vorgelegt und anschließend das Polymergebilde entweder gleichmäßig oder aber auf bestimmte Areale der Substratoberfläche aufgebracht, vorzugsweise gestreut wird. Anschließend werden dann die auf der Substratoberfläche befindlichen wasserabsorbierenden Polymergebilde mit der Feinstteilchenkomponente in Kontakt gebracht, beispielsweise dadurch, dass die Feinstteilchenkomponente auf die auf der Substratoberflächen befindlichen Polymergebilde gestreut werden. Schließlich erfolgt die Immobilisierung der Feinstteilchenkomponente auf der Oberfläche der Polymergebilde, wobei diese Immobilisierung vorzugsweise durch das vorstehend im Zusammenhang mit dem erfindungsgemäßen Verfahren zur Behandlung der Oberfläche wasserabsorbierender Polymergebilde beschriebene Erhitzen erfolgt. Bei dieser Variante der besonderen Ausgestaltung des erfindungsgemäßen Verfahrens zur Herstellung eines Verbundes erfolgt mithin der Verfahrensschritt V) nach dem Verfahrensschritt IV).

Gemäß einer anderen Variante dieser besonderen Ausgestaltung des erfindungsgemäßen Verfahrens zur Herstellung eines Verbundes wird zunächst das Substrat vorgelegt. Anschließend wird das unbehandelte, vorzugsweise jedoch bereits oberflächennachvernetzte, wasserabsorbierende Polymergebilde, welches zu mindestens 50 Gew.-% auf carboxylatgruppenhaltigen Monomeren basiert, mit dem Substrat in Kontakt gebracht, vorzugsweise dadurch, dass zunächst das Substrat vorgelegt und anschließend das Polymergebilde entweder gleichmäßig oder aber auf bestimmte Areale der Substratoberfläche aufgebracht, vorzugsweise gestreut wird. Noch bevor das Polymergebilde mit der Substratoberfläche in Kontakt gebracht wird, werden die wasserabsorbierenden Polymergebilde mit der Feinstteilchenkomponente in Kontakt gebracht, beispielsweise dadurch, dass die Feinstteilchenkomponente mit dem Polymergebilde, bevor es auf die Substratoberfläche gestreut wird, vermischt wird. Nach dem die Polymergebilde mit dem Substrat in Kontakt gebracht wurden, erfolg dann die Immobilisierung der Feinstteilchenkomponente auf der Oberfläche der Polymergebilde. Bei dieser Variante der besonderen Ausgestaltung des erfindungsgemäßen Verfahrens zur Herstellung eines Verbundes erfolgt mithin der Verfahrensschritt V) vor dem Verfahrensschritt IV).

Einen Beitrag zur Lösung der Eingangs genannten Aufgaben liefert auch ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

Einen weiteren Beitrag zur Lösung der Eingangs genannten Aufgaben liefern chemische Produkte beinhaltend die erfindungsgemäßen Polymergebilde, welche zu mindestens 50 Gew.-% auf carboxylatgruppenhaltigen Monomeren basieren, oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen Polymergebilde, welche zu mindestens 50 Gew.-% auf carboxylatgruppenhaltigen Monomeren basieren, oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierende Mittel oder Pflanzenschutzwirkstoffe liefern einen Beitrag zur Lösung der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Einen weiteren Beitrag zur Lösung der Eingangs genannten Aufgabe liefert die Verwendung einer Kombination aus einem wasserlöslichen Salz des Aluminiums und einem pulverförmigen Zinkoxid zur Behandlung der Oberfläche wasserabsorbierender Polymergebilde, vorzugsweise zur Verbesserung des Retentionsvermögens wasserabsorbierender Polymergebilde.

Die Erfindung wird nun anhand von nicht limitierenden Beispielen näher erläutert.

### BEISPIELE

### HERSTELLUNG VON SAP-PARTIKELN

Eine Monomerlösung bestehend aus 600 g Acrylsäure, die zu 70 Mol-% mit Natronlauge neutralisiert wurde (466,22 g 50%ige NaOH), 881,52 g Wasser, 0,573 g Polyethylenglykol-300-diacrylat, 1,603 g Monoallylpolyethylenglykol-450-monoacrylsäureester und 17,2 g Polyethylenglykol-750-monomethacrylsäure-estermethylether wird durch Spülen mit Stickstoff vom gelösten Sauerstoff befreit und auf die Starttemperatur von 4°C abgekühlt. Nach Erreichen der Starttemperatur wurde die Initiatorlösung (0,6 g Natriumperoxydisulfat in 10 g H₂O, 0,014 g 35%ge Wasserstoffperoxid-Lösung in 10 g H₂O und 0,03 g Ascorbinsäure in 10 g H₂0) zugesetzt. Nachdem die Endtemperatur von ca.100°C erreicht war, wurde das entstandene Gel mit einem Fleischwolf zerkleinert und bei 150°C 2 Stunden lang im Trockenschrank getrocknet. Das getrocknete Polymerisat wurde grob zerstoßen, mittels einer Schneidmühle SM 10 mit einem 2 mm-Sieb gemahlen und auf ein Pulver mit einer Partikelgröße von 150 bis 850 µm gesiebt (=Pulver A).

### VERGLEICHSBEISPIEL (herkömmliche Nachvernetzung)

100 g des Pulvers A werden mit einer Lösung bestehend aus 1,0 g Ethylencarbonat, 0,2 g Al₂(SO₄)₃×14 H₂O und 2,5 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver A in einem Umluftschrank bei 170°C für 45 Minuten erhitzt. Es wurde ein Pulver B erhalten.

### BEISPIEL 1

Eine Lösung aus 150 g entionisiertem Wasser, 12 g Al₂(SO₄)₃×14H₂O, 12 g Nanox^{®} 200 (Zinkoxidpulver der Firma Elementis Specialities, USA, mit einer BET-Oberfläche von 17 m²/g und einer mittleren Partikelgröße von 60 nm) und 60 g Ethylencarbonat wurde mittels eines Ultra Turax unter Bildung einer Dispersion vermischt. Mittels einer Spritze mit einer 0,9 mm-Kanüle werden 3,9 g dieser Suspension auf 100 g des sich in einem Mischer befindlichen Polymerisatpulvers A aufgetragen. Anschließend wurde das mit der Suspension beschichtete Pulver A in einem Umluftschrank bei 170°C für 45 Minuten erhitzt. Es wurde ein Pulver C erhalten. Das R/LA-Verhältnis betrug 2,01 g/g·%.

### BEISPIEL 2

Eine Lösung aus 150 g entionisiertem Wasser, 18 g Al₂(SO₄)₃×14 H₂O, 18 g Nanox^{®} 200 (Zinkoxidpulver der Firma Elementis Specialities, USA, mit einer BET-Oberfläche von 17 m²/g und einer mittleren Partikelgröße von 60 nm) und 60 g Ethylencarbonat wurde mittels eines Ultra Turax unter Bildung einer Dispersion vermischt. Mittels einer Spritze mit einer 0,9 mm-Kanüle werden 4,1 g dieser Suspension auf 100 g des sich in einem Mischer befindlichen Polymerisatpulvers A aufgetragen. Anschließend wurde das mit der Suspension beschichtete Pulver A in einem Umluftschrank bei 170°C für 45 Minuten erhitzt. Es wurde ein Pulver D erhalten. Das R/LA-Verhältnis betrug 2,05 g/g·%.

### BEISPIEL 3

Eine Lösung aus 150 g entionisiertem Wasser, 24 g Al₂(SO₄)₃×14 H₂O, 30 g Nanox^{®} 200 (Zinkoxidpulver der Firma Elementis Specialities, USA, mit einer BET-Oberfläche von 17 m²/g und einer mittleren Partikelgröße von 60 nm) und 60 g Ethylencarbonat wurde mittels eines Ultra Turax unter Bildung einer Dispersion vermischt. Mittels einer Spritze mit einer 0,9 mm-Kanüle werden 4,4 g dieser Suspension auf 100 g des sich in einem Mischer befindlichen Polymerisatpulvers A aufgetragen. Anschließend wurde das mit der Suspension beschichtete Pulver A in einem Umluftschrank bei 170°C für 45 Minuten erhitzt. Es wurde ein Pulver E erhalten. Das R/LA-Verhältnis betrug 2,00 g/g·%.

Die Polymerisatpulver weisen die folgenden Absorptionseigenschaften auf:

| Pulver | Beschichtung mit | | | |
|---|---|---|---|---|
| | Ethylencarbonat [Gew.-%] | Aluminiumsulfat [Gew.-%] | Nanox^{®} 200 [Gew.-%] | CRC-Wert [g/g] |
| A | 0 | 0 | 0 | 39,9 |
| B | 1,0 | 0,2 | 0 | 32,7 |
| C | 1,0 | 0,2 | 0,2 | 34,2 |
| D | 1,0 | 0,3 | 0,3 | 34,8 |
| E | 1,0 | 0,4 | 0,5 | 34,0 |

Wie den vorstehenden Absorptionswerten zu entnehmen ist, kann durch die erfindungsgemäße Oberflächenbehandlung der Polymergebilde mit dem Metallsalz (Aluminiumsulfat) und dem Oxid eines Metalls (Nanox^{®} 200) der bei der Oberflächennachvernetzung beobachtete Abfall des CRC-Wertes erkennbar vermindert werden.

### BEISPIEL 4

Die im Herstellungsbeispiel erhaltenen Polymere wurden, wie im Vergleichsbeispiel beschrieben, nachvernetzt, jedoch ohne Zusatz von Al₂(SO₄)₃×14 H₂O in der Nachvernetzerlösung. 100 g der so erhaltenen Polymere wurden im Trockenschrank auf 130°C vorgewärmt. Eine Mischung aus 24 g Al₂(SO₄)₃×14 H₂O, welches in einer Zentrifugalmühle gemahlen und auf eine Partikelgröße in einem Bereich von 300 bis 400 µm abgesiebt wurde, 30 g Nanox^{®} 200 (Zinkoxidpulver der Firma Elementis Specialities, USA, mit einer BET-Oberfläche von 17 m²/g und einer mittleren Partikelgröße von 60 nm) und 3,6 g Polyethylenglykol 10.000 (Polyethylenglykol mit einem Molekulargewicht von 10.000 g/mol), welches ebenfalls in einer Zentrifugalmühle gemahlen und auf eine Partikelgröße von weniger als 300 µm abgesiebt wurde), wurde hergestellt. 1,15 g dieser Mischung wurden in einem Krups-Mixer unter Rühren mit dem vorgewärmten wasserabsorbierenden Polymergebilde vermischt.

## Patentansprüche

1. Wasserabsorbierendes Polymergebilde, welches zu mindestens 50 Gew.-% auf carboxylatgruppenhaltigen Monomeren basiert und dessen Oberfläche mit einer Kombination aus einem wasserlöslichen Salz des Aluminiums und einem pulverförmigen Zinkoxid in Kontakt gebracht worden ist.

2. Wasserabsorbierendes Polymergebilde nach Anspruch 1, wobei das Polymergebilde ein R/LA-Verhältnis von mindestens 2,00 g/g·% aufweist.

3. Wasserabsorbierendes Polymergebilde nach Anspruch 1, wobei das wasserlösliche Salz des Aluminiums ein Sulfat ist.

4. Wasserabsorbierendes Polymergebilde nach einem der Ansprüche 1 bis 3, wobei mindestens 50 Gew.-% des Zinkoxids eine Partikelgröße in einem Bereich von 10 bis 1.000.000 nm aufweisen.

5. Wasserabsorbierendes Polymergebilde nach einem der vorhergehenden Ansprüche, wobei das Polymergebilde
- einen Innenbereich sowie
- einen den Innenbereich umgebenden Aussenbereich
aufweist und wobei der Aussenbereich einen höheren Vernetzungsgrad aufweist als der Innenbereich.

6. Verfahren zur Behandlung der Oberfläche wasserabsorbierender Polymergebilde, umfassend die Verfahrensschritte
i) Bereitstellen eines unbehandelten, wasserabsorbierenden Polymergebildes welches zu mindestens 50 Gew.-% auf carboxylatgruppenhaltigen Monomeren basiert;
ii) in Kontakt bringen der Oberfläche des unbehandelten, wasserabsorbierenden Polymergebildes mit einer Kombination aus einem wasserlöslichen Salz des Aluminiums und einem pulverförmigen Zinkoxid bei einer Temperatur in einem Bereich von 30 bis 300 °C.

7. Verfahren nach Anspruch 6, wobei das wasserlösliche Salz des Aluminiums und das pulverförmige Zinkoxidin Form eines Fluids F₁ umfassend ein Lösungsmittel, das wasserlösliche Salz des Aluminiums und das pulverförmige Zinkoxidmit dem wasserabsorbierenden Polymergebilde in Kontakt gebracht werden.

8. Verfahren nach Anspruch 7, wobei das Fluid F₁ zusätzlich einen Nachvernetzer, welcher in einer Kondensationsreaktion mit funktionellen Gruppen des wasserabsorbierenden Polymergebildes zu reagieren vermag, umfasst.

9. Verfahren nach einem der Ansprüche 6 bis 8, wobei das wasserlösliche Salz des Aluminiums in einer Menge in einem Bereich von 0,001 bis 5 Gew.-% und das pulverförmige Zinkoxid in einer Menge in einem Bereich von 0,001 bis 5 Gew.-%, jeweils bezogen auf das Gewicht des unbehandelten, wasserabsorbierenden Polymergebildes, mit dem unbehandelten wasserabsorbierenden Polymergebilde in Kontakt gebracht werden.

10. Verbund, beinhaltend ein wasserabsorbierendes Polymergebilde nach einem der Ansprüche 1 bis 5 und ein Substrat.

11. Verfahren zur Herstellung eines Verbundes, wobei ein wasserabsorbierendes Polymergebilde nach einem der Ansprüche 1 bis 5 und ein Substrat und gegebenenfalls ein Hilfsmittel miteinander in Kontakt gebracht werden.

12. Schäume, Formkörper, Fasern, Folien, Filme, Kabel, Dichtungsmaterialien, flüssigkeitsaufnehmende Hygieneartikel, Träger für pflanzen- und pilzwachstumregulierende Mittel, Verpackungsmaterialien, Bodenzusätze oder Baustoffe, beinhalten das wasserabsorbierende Polymergebilde nach einem der Ansprüche 1 bis 5 oder den Verbund nach Anspruch 10.

13. Verwendung des wasserabsorbierenden Polymergebildes nach einem der Ansprüche 1 bis 5 oder des Verbunds nach Anspruch 10 in Schäumen, Formkörpern, Fasern, Folien, Filmen, Kabeln, Dichtungsmaterialien, flüssigkeitsaufnehmenden Hygieneartikeln, Trägern für pflanzen- und pilzwachstumregulierenden Mitteln, Verpackungsmaterialien, Bodenzusätzen, zur kontrollierten Freisetzung von Wirkstoffen oder in Baustoffen.

14. Verwendung einer Kombination aus einem wasserlöslichen Salz des Aluminiumsund pulverförmigen Zinkoxidzur Behandlung der Oberfläche superabsorbierender Polymergebilde, welche zu mindestens 50 Gew.-% auf carboxylatgruppenhaltigen Monomeren basieren.

## Claims

1. Water-absorbing polymer structure which is based to an extent of at least 50% by weight on monomers containing carboxylate groups and has been surface contacted with a combination of a water-soluble salt of aluminium and pulverulent zinc oxide.

2. Water-absorbing polymer structure according to Claim 1, wherein the polymer structure has an R/E ratio of at least 2.00 g/g·%.

3. Water-absorbing polymer structure according to Claim 1, wherein the water-soluble salt of aluminium is a sulphate.

4. Water-absorbing polymer structure according to any of Claims 1 to 3, wherein at least 50% by weight of the zinc oxide has a particle size within a range from 10 to 1 000 000 nm.

5. Water-absorbing polymer structure according to any of the preceding claims, wherein the polymer structure has
- an inner region and
- an outer region surrounding the inner region
and wherein the outer region has a higher crosslinking level than the inner region.

6. Process for treating the surface of water-absorbing polymer structures, comprising the process steps of:
i) providing an untreated water-absorbing polymer structure based to an extent of at least 50% by weight on monomers containing carboxylate groups;
ii) contacting the surface of the untreated water-absorbing polymer structure with a combination of a water-soluble salt of aluminium and a pulverulent zinc oxide at a temperature within a range from 30 to 300°C.

7. Process according to Claim 6, wherein the water-soluble salt of aluminium and the pulverulent zinc oxide are contacted with the water-absorbing polymer structure in the form of a fluid F₁ comprising a solvent, the water-soluble salt of aluminium and the pulverulent zinc oxide.

8. Process according to Claim 7, wherein the fluid F₁ additionally comprises a postcrosslinker capable of reacting with functional groups of the water-absorbing polymer structure in a condensation reaction.

9. Process according to any of Claims 6 to 8, wherein the water-soluble salt of aluminium is contacted with the untreated water-absorbing polymer structure in an amount within a range from 0.001% to 5% by weight and the pulverulent zinc oxide in an amount within a range from 0.001% to 5% by weight, based in each case on the weight of the untreated water-absorbing polymer structure.

10. Composite comprising a water-absorbing polymer structure according to any of Claims 1 to 5 and a substrate.

11. Process for producing a composite, wherein a water-absorbing polymer structure according to any of Claims 1 to 5 and a substrate and optionally an assistant are contacted with one another.

12. Foams, mouldings, fibres, foils, films, cables, sealing materials, liquid-absorbing hygiene articles, carriers for plant growth and fungal growth regulators, packaging materials, soil additives or building materials, comprising the water-absorbing polymer structure according to any of Claims 1 to 5 or the composite according to Claim 10.

13. Use of the water-absorbing polymer structure according to any of Claims 1 to 5 or of the composite according to Claim 10 in foams, mouldings, fibres, foils, films, cables, sealing materials, liquid-absorbing hygiene articles, carriers for plant growth and fungal growth regulators, packaging materials and soil additives, for controlled release of active ingredients or in building materials.

14. Use of a combination of pulverulent zinc oxide and a water-soluble salt of aluminium for treatment of the surface of superabsorbent polymer structures based to an extent of at least 50% by weight on monomers containing carboxylate groups.

## Revendications

1. Entité polymère absorbant l'eau, qui est à raison d'au moins 50 % en poids à base de monomères contenant des groupes carboxylate et dont la surface a été mise en contact avec une association d'un sel hydrosoluble de l'aluminium et d'un oxyde de zinc pulvérulent.

2. Entité polymère absorbant l'eau selon la revendication 1, l'entité polymère présentant un rapport R/LA d'au moins 2,00 % g/g.

3. Entité polymère absorbant l'eau selon la revendication 1, dans laquelle le sel hydrosoluble de l'aluminium est un sulfate.

4. Entité polymère absorbant l'eau selon l'une quelconque des revendications 1 à 3, dans laquelle au moins 50 % en poids de l'oxyde de zinc présentent une taille de particule dans une plage de 10 à 1 000 000 nm.

5. Entité polymère absorbant l'eau selon l'une quelconque des revendications précédentes, l'entité polymère comportant
- une partie interne ainsi
- qu'une partie externe entourant la partie interne et la partie externe présentant un degré de réticulation plus élevé que la partie interne.

6. Procédé pour le traitement de la surface d'entités polymères absorbant l'eau, comprenant les étapes de processus
i) disposition d'une entité polymère qui est à raison d'au moins 50 % en poids à base de monomères contenant des groupes carboxylate ;
ii) mise en contact de la surface de l'entité polymère absorbant l'eau, non traitée, avec une association d'un sel hydrosoluble de l'aluminium et d'un oxyde de zinc pulvérulent, à une température dans une plage de 30 à 300 °C.

7. Procédé selon la revendication 6, dans lequel on met en contact avec l'entité polymère absorbant l'eau le sel hydrosoluble de l'aluminium et l'oxyde de zinc pulvérulent sous la forme d'un fluide F₁ comprenant un solvant, le sel hydrosoluble de l'aluminium et l'oxyde de zinc pulvérulent.

8. Procédé selon la revendication 7, dans lequel le fluide F₁ comprend en outre un agent de post-réticulation qui a la capacité de réagir avec des groupes fonctionnels de l'entité polymère absorbant l'eau.

9. Procédé selon l'une quelconque des revendications 6 à 8, dans lequel on met en contact avec l'entité polymère absorbant l'eau, non traitée, le sel hydrosoluble de l'aluminium en une quantité dans une plage de 0,001 à 5 % en poids et l'oxyde de zinc pulvérulent en une quantité dans une plage de 0,001 à 5 % en poids, chaque fois par rapport au poids de l'entité polymère absorbant l'eau, non traitée.

10. Composite, comportant une entité polymère absorbant l'eau selon l'une quelconque des revendications 1 à 5 et un support.

11. Procédé pour la production d'un composite, dans lequel on met en contact entre eux une entité polymère absorbant l'eau selon l'une quelconque des revendications 1 à 5 et un support et éventuellement un adjuvant.

12. Mousses, corps moulés, fibres, feuilles, films, câbles, matériaux d'étanchéité, articles d'hygiène absorbant les liquides, supports pour agents régulateurs de croissance de plantes et de champignons, matériaux d'emballage, additifs pour sols ou matériaux de construction, comportant l'entité polymère absorbant l'eau selon l'une quelconque des revendications 1 à 5 ou le composite selon la revendication 10.

13. Utilisation de l'entité polymère absorbant l'eau selon l'une quelconque des revendications 1 à 5 ou du composite selon la revendication 10 dans des mousses, corps moulés, fibres, feuilles, films, câbles, matériaux d'étanchéité, articles d'hygiène absorbant les liquides, supports pour agents régulateurs de croissance de plantes et de champignons, matériaux d'emballage, additifs pour sols, pour la libération programmée de substances actives ou dans des matériaux de construction.

14. Utilisation d'une association d'un sel hydrosoluble de l'aluminium et d'oxyde de zinc pulvérulent pour le traitement de la surface d'entités polymères absorbant l'eau qui sont à raison d'au moins 50 % en poids à base de monomères contenant des groupes carboxylate.
